(19) Europäisches Patentamt
European Patent Office
Office européen des brevets

(11) Veröffentlichungsnummer: **0 246 446 B1**

# (12) EUROPÄISCHE PATENTSCHRIFT

(45) Veröffentlichungstag der Patentschrift: **19.12.90**

(51) Int. Cl.[5]: **C08F 291/00**, C08F 257/02, G01N 33/545, C07K 17/08 // (C08F257/02, 220:58)

(21) Anmeldenummer: **87105536.4**

(22) Anmeldetag: **14.04.87**

## (54) Dispersionspolymere, Verfahren zu ihrer Herstellung und ihre Verwendung.

(30) Priorität: **18.04.86 DE 3613111**

(43) Veröffentlichungstag der Anmeldung: **25.11.87 Patentblatt 87/48**

(45) Bekanntmachung des Hinweises auf die Patenterteilung: **19.12.90 Patentblatt 90/51**

(84) Benannte Vertragsstaaten: **AT BE CH DE ES FR GB IT LI LU NL SE**

(56) Entgegenhaltungen:
**EP-A- 0 080 614**
**GB-A- 1 469 358**

(73) Patentinhaber: **BEHRINGWERKE Aktiengesellschaft, Postfach 1140, D-3550 Marburg 1(DE)**

(72) Erfinder: **Kapmeyer, Wolfgang, Dr., Reinhardswaldstr. 5, D-3550 Marburg(DE)**

(74) Vertreter: **Klein, Otto, Dr. et al, Hoechst AG Zentrale Patentabteilung Postfach 80 03 20, D-6230 Frankfurt am Main 80(DE)**

EP 0 246 446 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft Dispersionspolymere, Verfahren zu ihrer Herstellung und ihre Verwendung, wobei die Dispersionspolymere aus Latexpartikeln bestehen, die nach einem Saatpolymerisationsverfahren derart hergestellt worden sind, daß das eingesetzte Saatpolymerisat noch einen Anteil von 5 bis 50 Gew. % nicht auspolymerisierter Monomere enthält und die Saatpolymerisation mit Monomeren durchgeführt wird, wobei mindestens ein Monomer eine Acetalgruppen enthaltende Verbindung ist.

Man gewinnt daraus biologisch aktive Dispersionspolymere, indem man biologisch aktive Substanzen, die über freie Aminogruppen verfügen, an reaktive, von der Aldehydfunktion abgeleitete Gruppen auf der Oberfläche der erfindungsgemäßen Dispersionspolymerteilchen bindet. Diese biologisch aktiven Latexkonjugate sind geeignet für serologische oder immunologische Bestimmungsverfahren.

Es ist bekannt, die Empfindlichkeit serologischer oder immunologischer Bestimmungsverfahren durch die Verwendung von Indikator- oder Trägerteilchen zu erhöhen, die mit dem entsprechenden immunologischen Reagenz beladen sind. Als Trägermaterial können z.B. rote Blutkörperchen oder Zellen einer Zellkultur verwendet werden. Auch Latexpartikel mit einem Durchmesser von 0,02 bis 5 µm werden hierfür eingesetzt.

Aus der Europäischen Patentanmeldung EP-A 0 080 614 sind Latexpartikel bekannt, die über Säureamidgruppen gebundene Acetal-Funktionen enthalten. In einem wässrigen Medium vorgefertigte Latexkerne werden mit Vinylmonomeren, die über Säureamidgruppen gebundene Acetal-Funktionen enthalten, angequollen und diese Vinylmonomere, die ausreichend wasserunlöslich sein müssen, werden dann zusammen mit weiteren Monomeren, die hydrophiler oder ionischer Natur sein können, copolymerisiert. Derartige Reagenzien lassen sich zur nephelometrischen und turbidimetrischen Bestimmung von Proteinen einsetzen.

Zu diesem Zweck werden Antikörper gegen das zu bestimmende Protein an den Latex gebunden. Nach entsprechender Verdünnung des antikörperbeladenen Latex kann das so entstandene Reagenz zur Messung eingesetzt werden: Nach Inkubation mit dem zu bestimmenden Antigen bilden sich Agglutinate, die nephelometrisch oder turbidimetrisch gemessen werden können. Die erhaltenen Streulichtsignale nach diesem Stand der Technik sind recht niedrig und erlauben daher keine gut reproduzierbare Messung. Eine Steigerung der Nachweisempfindlichkeit des Reagenzes und damit verbunden auch eine Erhöhung der Streulichtsignale während der Umsetzung zwischen einem Reagenz nach dem Stand der Technik und dem nachzuweisenden Antigen ist nicht ohne weiteres möglich.

Es wurde nun überraschend gefunden, daß die geschilderten Nachteile des Standes der Technik überwunden werden können, wenn man Trägerpartikel verwendet, die nach einer Methode hergestellt sind, die dadurch gekennzeichnet ist, daß man vorgefertigte, nicht vollständig auspolymerisierte Latexkerne verwendet, bei denen noch ein beträchtlicher Anteil der eingesetzten Monomeren nicht polymerisiert ist, die also einen hohen Restmonomergehalt aufweisen, und diese nicht vollständig auspolymerisierten, und somit im Monomer, z.B. Styrol, gequollenen Latexkerne in einem wässrigen Medium mit Acryl- oder Methacrylmonomeren, die über Säureamidgruppen gebundene Acetalgruppen enthalten, gegebenenfalls zusammen mit Acryl- oder Methacrylsäuremonomeren, copolymerisiert.

Gegenstand der Erfindung sind somit Dispersionen, die Trägerteilchen aus Latex enthalten, die nach dem Saatdispersionsverfahren derart hergestellt worden sind, daß in dem verwendeten Saatlatex nur 30-90 Gew. %, vorzugsweise 40-80 Gew. %, der vorgegebenen Monomeren auspolymerisiert wurden, und auf deren Oberfläche ein Copolymerisat sich befindet, das aus einer Monomerenmischung polymerisiert wurde, die enthält Monomere der Formel I

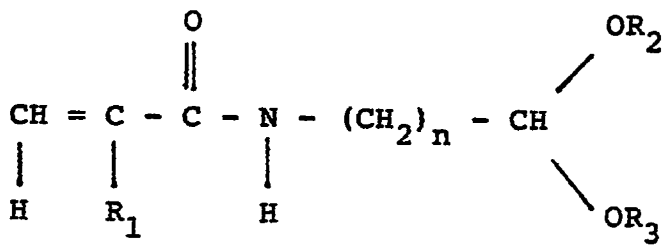

Formel I

worin n = 1 - 6;
$R_1$ = H, $CH_3$ und
$R_2$ und $R_3$ gleich oder unterschiedlich sind mit
$R_2$, $R_3$ = $-(CH_2)_m-CH_3$, m = 0 - 7 oder

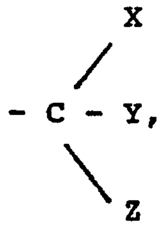, wobei X, Y, Z = $(CH_2)_pCH_3$ und p = 1 - 3 sind, mit X, Y, Z gleich oder unterschiedlich

oder $R_2$, $R_3$ = Arylrest

und gegebenenfalls Croton- und/oder Acryl- und/oder Methacrylsäure sowie gegebenenfalls das Monomer, aus dem die Saatpolymere gebildet sind, also z.B. Styrol.

Gegenstand der Erfindung ist ferner ein Verfahren zur Herstellung der Dispersionen, das dadurch gekennzeichnet ist, daß man zunächst eine Latexdispersionssaat herstellt, indem man Monomere, wie z.B. Styrol, so polymerisiert, daß nur 30-90 Gew. % der eingesetzten Monomeren auspolymerisiert werden und danach das überschüssige Monomer, das nicht im Polymeren gelöst ist, durch Filtration oder durch ausgiebige Dialyse abtrennt und sodann eine Monomerenmischung zugibt und aufpolymerisiert. Diese Mischung enthält Verbindungen mit endständigen Acetalen der Formel I und gegebenenfalls Croton- und/oder Acryl- und/oder Methacrylmonomere sowie Monomere, aus denen das Saatpolymer gebildet ist, z.B. Styrol. Vorzugsweise wird der Saatlatex so polymerisiert, daß nur 40-80 Gew. % der vorgelegten Monomeren auspolymerisiert wurden. Besonders bevorzugt ist ein Polymerisationsverfahren für den Saatlatex, für das 50-70 Gew. % der vorgegebenen Monomeren auspolymerisiert wurden.

Nach dem erfindungsgemäßen Verfahren erhält man somit Polymerkerne, die noch 5-50 Gew. % Monomeres, bezogen auf das Polymer, enthalten. Bevorzugt sind Präparationen, die 10-40 Gew. % Monomeres, bezogen auf Polymeres, enthalten. Besonders bevorzugt sind Präparationen, die 20-30 Gew. % Monomeres, bezogen auf Polymeres, enthalten.

Die Latexpartikel, die als Saatdispersion für die erfindungsgemäßen Dispersionen eingesetzt werden, sollen nicht-filmbildende Polymerisate sein. Unter "nicht-filmbildend" werden Polymer-Latexteilchen verstanden, die keinen Film bilden und unter den Anwendungsbedingungen nicht zusammenfließen. Polymerisate aus carbocyclischen aromatischen Monovinylidenmonomeren, wie Styrol, Vinyltoluol und Vinylnaphthalin sowie Mischungen dieser Monomeren untereinander und/oder mit Methylmethacrylat und Acrylnitril sind bevorzugt. Besonders bevorzugte Saatdispersionen sind Polystyrol-Latices. Die Herstellung einer Polystyrolsaatdispersion erfolgt nach an sich bekannten Verfahren.

Zur Herstellung des erfindungsgemäßen Dispersionspolymeren wird grundsätzlich ein vorgefertigter Latex mit Partikeldurchmesser von 0,02 bis 2 µm, vorzugsweise 0,05 his 0,5 µm, mit etwa 20-80 Gew. % der Menge eines Emulgators versetzt, der zur maximalen monomolekularen Bedeckung der Latexoberfläche notwendig wäre. Messungen zur Bestimmung der Emulgatormenge, die zu einer maximalen Bedeckung der Latexoberfläche führt, werden mit Hilfe eines Tensiometers durchgeführt. Sie sind zum Beispiel von I. Phrma und S.-R. Chen im Journal of Colloid and Interface Science, Vol. 74 (1979), S. 90-102 und erstmalig von S.H. Maron, M.E. Elder und I.N. Ulevitch im Journal of Colloid Interface Sciences, Vol. 9 (1954), S. 89-104, publiziert worden.

In Frage kommende Emulgatoren sind beispielsweise Polyglycolether mit langkettigen, aliphatischen Alkoholen, die vorzugsweise 10-20 Kohlenstoffatome aufweisen, oder Alkylphenol, deren Alkylrest vorzugsweise 6-12 Kohlenstoffatome enthält, oder Dialkylphenole oder Trialkylphenole, deren Alkylreste vorzugsweise verzweigte Alkylreste mit jeweils 3-12 Kohlenstoffatomen darstellen. Beispiele hierfür sind Umsetzungsprodukte von Äthylenoxyd mit Laurylalkohol, Stearylalkohol, Oleylalkohol, Kokosfettalkohol, Octylphenol, Nonylphenol, Diisopropylphenol, Triisopropylphenol, Di-t-butylphenol und Tri-t-butylphenol. Reaktionsprodukte des Ethylenoxids mit Polypropylenglykol sind ebenfalls geeignet.

Von den ionischen Emulgatoren sind vor allem anionische Emulgatoren geeignet, insbesondere A lkali- oder Ammoniumsalze von Alkylsulfonaten oder Alkylarylsulfonaten sowie von den entsprechenden Sulfaten, Phosphaten oder Phosphonaten, die gegebenenfalls Oxyethylen-Einheiten zwischen dem jeweiligen Kohlenwasserstoffrest und der anionischen Gruppe aufweisen. Beispiele hierfür sind Natriumdodecylsulfat, Natriumlaurylsulfat, Natriumoctylphenolglykolethersulfat, Natriumdodecylbenzolsulfonat, Natriumlauryldiglykolsulfat, Ammonium-tri-t-butylphenolpentaglykolsulfat und Ammonium-tri-t-butylphenoloctaglykolsulfat. Bevorzugt eingesetzt wird Natriumdodecylsulfat.

Zu der Saatdispersion, die neben Emulgator einen Radikalstarter (Inititator) enthält, wird ein Monomerengemisch, das mindestens eine Acetalgruppen enthaltende Verbindung der Formel I enthält, unter Rühren zugetropft. Die Temperatur der Dispersion liegt zwischen + 10° C und + 120° C, vorzugsweise zwischen + 50° C und + 90° C.

Die Polymerisation erfolgt nach an sich bekannten Verfahren in Gegenwart eines radikalbildenden Initiators, z.B. einer Peroxid-Verbindung oder einer aliphatischen Azoverbindung. Der Initiator ist wasserlöslich; er wird in einer Menge von 0,05 bis 10 Gew. %, vorzugsweise 0,1 bis 3 Gew. % eingesetzt (bezogen auf die Gesamtmenge der Monomeren). Bekannte radikalbildende Initiatoren sind zum Beispiel Wasserstoffperoxid, Alkali- oder Ammoniumsalze der Peroxydischwefelsäure oder der Peroxydiphosphorsäure, zum Beispiel Natriumperoxydisulfat, Kaliumperoxydisulfat und Ammoniumperoxydisulfat, ferner Alkylhydroperoxide wie t-Butylhydroperoxid, Dialkylperoxide wie Di-t-butylperoxid, Diacylperoxide wie Diacetylperoxid, Dilauroylperoxid und Dibenzoylperoxid sowie Azodiisobuttersäurenitril, Azodicarbonamid und Azo-gamma,gamma'-bis(4-cyanvaleriansäure). Bevorzugt eingesetzt werden die Alkali- oder Ammoniumsalze der Peroxidischwefelsäure, wie Natrium-, Kalium- und Ammoniumperoxidisulfat.

Der Initiator wird gegebenenfalls zusammen mit einem Reduktionsmittel eingesetzt, insbesondere mit einem Alkalisalz oder Erdalkalisalz einer reduzierend wirkenden schwefelhaltigen Säure; vorzugsweise eignen sich Sulfite, Bisulfite, Pyrosulfite, Dithionite, Thiosulfate und Formaldehydsulfoxylate. Gleichfalls verwendbar sind auch Glukose und Ascorbinsäure.

Als Acetalgruppen enthaltende Monomere werden die Verbindungen der Formel I eingesetzt, bevorzugt verwendet man Acryl- oder Methacrylamidoalkylaldehyd-di-alkylacetal mit Alkyl = $C_2$ bis $C_8$. Ganz besonders geeignet sind Acryl- oder Methacrylamidoacetaldehyd-di-n-pentylacetal.

Dem eine Verbindung der Formel I enthaltenden Monomerengemisch können bis zu 30 Gew. %, bezogen auf die Gesamtmischung, Styrol, Vinylnaphthalin oder Vinyltoluol zugegeben werden. Außerdem kann das Monomerengemisch gegebenenfalls zusätzlich bis zu 30 Gew. %, bezogen auf die Gesamtmischung, Methacrylsäure, Acrylsäure oder Crotonsäure enthalten.

Der Saatdispersion wird das Monomerengemisch zugegeben in Mengen von 90 bis 5 Gew. %; vorzugsweise 40 bis 10 Gew. %, bezogen auf die Gesamtmenge von Saatdispersion und Monomerenmischung.

Wichtig für das Gelingen der Polymerisation in Anwesenheit des Saatpolomeren kann es sein, daß die Monomerenmischung unter ständigem Rühren der Suspension der Latexkerne bei Polymerisationsbedingungen, d.h. bei einer Temperatur von + 10° C bis + 120° C, vorzugsweise + 50° C bis + 90° C, zugetropft wird. Während des Zutropfens zieht ständig die hinzugegebene Menge der Monomere auf die schon fertigen Saatlatexpartikel auf, wird dort aufpolymerisiert und bildet weiteres Polymeres um den Latexpartikel.

Anschließend wird das Polymerisat von überschüssigen Monomeren, Resten von Initiator und Emulgator nach bekannten Verfahren befreit. Vorteilhafterweise unterwirft man das Polymerisat einer Dialyse, zum Beispiel gegen $NaHCO_3$-Puffer (0,01 bis 0,05 Gew. %).

Zur Herstellung der erfindungsgemäßen, biologisch aktiven Dispersionen, nachfolgend auch als Latexkonjugat bezeichnet, wird eine Suspension der oben beschriebenen saatpolymerisierten Latexpartikel auf einen pH-Wert unter 5, vorzugsweise unter 3, eingestellt und mit dem zu bindenden immunologisch aktiven Material, wie zum Beispiel Antikörper, Antigene oder Haptene, inkubiert.

Die labilen Bindungen zwischen einer Aminogruppe des Proteins und dem freigesetzten Aldehyd auf dem erfindungsgemäßen Latexpartikel werden nach bekannten Verfahren reduziert. Bevorzugt wird eine Lösung von Natriumcyanborhydrid in neutralem Puffer hierfür verwendet. Man trennt eventuell ungebundenes immunologisch aktives Material oder andere Verunreinigungen aus dem Reaktionsansatz ab. Dies geschieht zweckmäßigerweise durch Zentrifugieren oder Waschen auf geeigneten Membranen.

Die erfindungsgemäßen, saatpolymerisierten Latices zeichnen sich durch besondere Stabilität aus. Sie eignen sich zur Herstellung empfindlicher Reagenzien. Die erfindungsgemäßen Reagenzien können durch Lyophilisation getrocknet werden.

Reagenzien, hergestellt nach dem Stand der Technik, zeigen bei nephelometrischer oder turbidimetrischer Messung eine Reaktion mit dem nachzuweisenden Antigen unter Agglutination und somit Erhöhung des Streulichtsignals bzw. der Extinktion in der Meßküvette. So wurde z.B. ein Reagenz zur Bestimmung von C-reaktivem Protein, einem wichtigen Entzündungsparameter, unter Verwendung von Kaninchen-Antikörpern gegen C-reaktives Protein (CRP) hergestellt. Wird dieses Reagenz mit unterschiedlichen CRP-Konzentrationen umgesetzt und die Streulichtsignale in einem Nephelometer nach 30 min. Inkubationszeit gemessen, so erhält man eine Standardkurve, die mit zunehmender CRP-Konzentration höhere Streulichtsignale zeigt. Eine derartige Standardkurve ist in Fig. 1 dargestellt. Man sieht deutlich, daß für die eingesetzten Konzentrationen an CRP nur niedrige Streulichtsignale gemessen werden können. Damit im Zusammenhang steht eine geringe Empfindlichkeit der Messung, die untere Nachweisgrenze des Reagenzes liegt bei ca. 288 µg/l CRP; d.h., CRP-Konzentrationen im Bereich zwischen 36 µg/l und 288 µg/l können nicht mehr erfaßt werden.

Das erfindungsgemäße Reagenz, hergestellt durch Bindung von Antikörpern beispielsweise gegen CRP an Latexpräparationen, gewonnen durch Saatpolymerisation auf nicht auspolymerisierten, Restmonomeren enthaltenden Polymeren, zeigt dagegen eine viel stärkere Agglutinationsreaktion nach Umsetzung mit CRP. Wird dieses erfindungsgemäße Reagenz mit unterschiedlichen CRP-Konzentrationen umgesetzt und werden nach 30 min. Inkubationszeit die Streulichtsignale in einem Nephelometer gemessen, so erhält man eine Standardkurve, die schon bei niedrigen CRP-Konzentrationen hohe Streulichtsignale zeigt. Eine derartige Standardkurve ist in Fig. 2 dargestellt. Man sieht, daß die Empfindlichkeit des Reagenzes so hoch ist, daß auch niedrige CRP-Konzentrationen bis 36 µg/l gemessen werden können. Hin-

zu kommt, daß die Streulichtsignale für CRP-Konzentrationen zwischen 36 und 2300 µg/l sehr viel höher sind als für ein Reagenz nach dem Stand der Technik. Das heißt, die Reproduzierbarkeit der Messung ist sehr viel besser als nach dem Stand der Technik.

Weitere erfindungsgemäße Reagenzien mit entsprechenden vorteilhaften Eigenschaften erhält man, wenn die erfindungsgemäßen Latexpräparationen beispielsweise mit Antikörpern gegen alpha-Fetoprotein (AFP), Ferritin, Human Placental Lactogen (HPL), Thyroxin bindendes Globulin (TBG), Immunglobulin E, $\beta_2$-Microglobulin, Schwangerschaft spezifisches $\beta_1$-Glycoprotein und Human chorionic Gonadotropin (βHCG), beladen werden.

Auch monoklonale Antikörper lassen sich zur Herstellung der erfindungsgemäßen Reagenzien vorteilhaft einsetzen. Gleichermaßen können Latexpräparationen mit bakteriellen oder viralen Proteinen wie z.B. Streptolysin O, Streptococcus B Antigen, H. Influenza Antigen, Pneumococcus Antigen, Lues Antigen, Toxoplasmen Antigenen, HBsAg, Rubella Antigen, Herpes Antigen und Tetanus Antigen beladen werden und die entsprechenden Antikörper durch diese Antigen beladenen, erfindungsgemäßen Reagenzien nachgewiesen werden.

Schließlich können in gleicher Weise Latexpräparationen gemäß der Erfindung mit derivatisierten Haptenen, z.B. Hormonen oder Arzneimitteln, wie Thyroxin ($T_4$), Trijodothyronin ($T_3$), Cortisol, Progesteron, Testosteron, Gentamycin und Digoxin beladen werden, wodurch man biologisch aktive Dispersionspolymere, also die erfindungsgemäßen Reagenzien, erhält, mit denen man durch Inhibitionsteste, also unter gleichzeitiger Verwendung geeigneter Antikörper, die Konzentration der genannten Haptene messen kann.

Die erfindungsgemäßen Latexpräparationen sind einfach herzustellen und schonend mit empfindlichen immunologisch aktiven Materialien zu einem diagnostischen Reagenz zu verknüpfen.

Die Latexkonjugate können in allen diagnostischen Verfahren eingesetzt werden, die Teilchengrößenveränderungen messen, beispielsweise in qualitativen und semiquantitativen Bestimmungen von Substanzen mit Hilfe von visuellen Latexagglutinationstesten sowie in nephelometrischen oder turbidimetrischen Bestimmungen von Spurenproteinen im direkten oder kompetitiven Agglutinationstest oder im Latex-Hapten-Inhibitionstest, oder im Partikelzählverfahren.

## Beispiele

### 1. Herstellung von Saatpolymerisat

In ein zylindrisches Glasgefäß, ausgestattet mit Gasein- und Gasauslaßrohr sowie einem Magnetrührstab wurden 310 ml stickstoffgesättigtes bidestilliertes Wasser gegeben. Man gab 500 mg Natriumstearat hinzu und löste dieses durch Rühren. Weiterhin wurden 1,5 ml Ammoniak, 25 %ig hinzugegeben. Der pH wurde kontrolliert und betrug 11,09. Durch mehrfaches Evakuieren und Füllung mit Stickstoff wurde das Polymerisationsgefäß sauerstoffrei gemacht. Unter ständigem Rühren wurde die Detergenz-Lösung mit Hilfe eines Wasserbades auf + 70° C erwärmt.

Anschließend gab man 90 ml frisch destilliertes Styrol unter Stickstoff mit Hilfe eines Tropftrichters mit Druckausgleich in das Polymerisationsgefäß. Bei + 70° C wurde 15 min. zur Emulgation des Styrols gerührt. Anschließend wurde die Temperature auf + 90° C angehoben und eine weitere Stunde gerührt. Danach wurden 67,5 mg Kaliumperoxydisulfat gelöst in 50 ml stickstoffgesättigtem destilliertem Wasser hinzugegeben. Man ließ 80 min. bei + 90° C rühren. Das Polystyrol wurde durch ein Faltenfilter gegeben. Hierbei bleiben unter Umständen einige ml Styrol auf dem Filter zurück. Dieses Styrol konnte, da etwas im Überschuß vorhanden, nicht vollständig im Polystyrol gelöst werden.

Das filtrierte Polystyrol wurde gegen 10 l 0,01 Gew. %ige Ammoniumhydrogencarbonat-Lösung (mit 0,01 Gew. % $NH_4HCO_3$; 0,01 Gew. % $NaN_3$; mit 10,5 ml Ammoniak 25 Gew. % in 10 l auf pH 10,0 eingestellt) 50 Stunden lang dialysiert. Man erhielt nach Dialyse 410 ml Polymerisat mit einemTrockengewicht von 12,8 g/dl. Somit sind etwa 60 Gew. % des eingesetzten Monomeren polymerisiert worden. Durch eine Verlängerung der Polymerisationszeit um 5 bis 10 min. ließ sich der Anteil des polymerisierten Styrols erhöhen, bei gleichzeitiger Reduktion des im Polystyrol gelösten Monomeren.

Durch eine Reduktion der Polymerisationszeit um 5 bis 10 min. ließ sich dagegen der Anteil des auspolymerisierten Styrols reduzieren, bei gleichzeitiger Erhöhung des im Styrol gelösten Monomeren.

### 2. Saatpolymerisation unter Verwendung des nach Beispiel 1 hergestellten Polymeren

In ein zylindrisches Glasgefäß, ausgestattet mit Gasein- und Gasauslaßrohr sowie einem Magnetrührstab wurden 156,3 ml einer Polystyrol-Latex-Dispersion mit einem Feststoffgehalt von 12,8 Gew. % sowie 238,7 ml destilliertes Wasser und 250 mg Natriumdodecylsulfat gegeben und durch Rühren gelöst. Durch mehrfaches Evakuieren und Füllen mit Stickstoff wurde das Polymerisationsgefäß sauerstoffrei gemacht. die Latex-Detergenzmischung wurde unter ständigem Rühren auf + 70° C in einem Wasserbad erhitzt.

Es wurde eine Monomerenmischung , hergestellt aus 2 ml Styrol, 2 ml Methacrylamidoacetaldehyd-di-n-pentylacetal und 1 ml Methacrylsäure unter Stickstoff hinzugegeben. Anschließend ließ man unter

Rühren 1 Stunde bei ca. 20° C emulgieren.

Dann wurde der Polymerisationsansatz im Wasserbad auf + 70° C erwärmt. Nach 15 min. Rühren bei + 70° C wurde die Copolymerisation gestartet. Hierzu gab man 5 ml einer Kaliumperoxydisulfatlösung (16 mg/ml in destilliertem Wasser) hinzu. Die Temperatur des Polymerisationsansatzes wurde auf + 70° C gehalten. Es wurde 5 Stunden bei der genannten Temperatur gerührt. Die Polymerisation war damit beendet und die Dispersion wurde auf Raumtemperatur abgekühlt und mittels Faltenfilter filtriert. Man erhielt 395 ml einer Latex-Suspension. Diese wurde anschließend 17 Stunden lang gegen eine $NaHCO_3$-Pufferlösung (0,25 g/l, pH 8-8,2) dialysiert. Man erhielt 415 ml einer Latex-Dispersion mit einem Feststoffgehalt von 6,8 Gew. %.

3. Bindung von Anti-CRP-Antikörpern an ein erfindungsgemäßes Polymerisat

An ein Polymerisat, hergestellt nach Beispiel 2, wurden Anti-CRP-Antikörper gebunden. Das verwendete Polymerisat wurde mit destilliertem Wasser auf einen Feststoffgehalt von 5,8 Gew. % verdünnt. Ein Antiserum, gewonnen durch Immunisierung von Kaninchen mit aufgereinigtem CRP wurde nach bekannten Verfahren als Gammafraktion isoliert. Es wurde anschließend ankonzentriert, bis ein Proteingehalt von 10 mg/ml erreicht war.

0,5 ml des obengenannten Polymerisates wurden mit 0,05 ml der Anti-CRP-Antikörper-Lösung gemischt. Dann wurden 0,025 ml einer 20 %igen wässrigen Lösung von Eikosaoxyethylen-sorbitanlaurylsäureester (Tween® 20) hinzugegeben und das Ganze nochmals gemischt. Hierzu gab man 0,01 ml 1 N HCl, so daß ein pH-Wert von ca. 2 erreicht wurde. Nach einer Inkubationszeit von 30 min. bei Raumtemperatur setzte man 0,125 ml gesättigte wässrige Natriumhydrogenphosphat-Lösung (pH 6,5) und 0,125 ml wässrige Natriumcyanborhydrid-Lösung (25 mg/ml) hinzu und mischte gut durch. Anschließend erfolgte eine Inkubation von einer Stunde bei Raumtemperatur.

Dieser Beladungsansatz wurde sodann 30 min. mit ca. 50.000 g zentrifugiert (Beckman Zentrifuge, 20.000 UpM). Der Überstand wurde verworfen. Der Rückstand wurde in 0,75 ml eines Glycin-NaCl-Puffers (0,1 Mol Glycin, 0,17 Mol NaCl, 0,5 Gew. % Eikosaoxyethylen-sorbitanlaurylsäureester (Tween® 20), pH 8,2) resuspendiert.

Anschließend erfolgte eine Ultraschall-Behandlung (Bronson Sonyfier B 15) für 2 Sekunden. Das so redispergierte Reagenz wurde im Volumenverhältnis 1:80 mit dem zuvor genannten Glycin-NaCl-Puffer verdünnt und nochmals für 30 Sekunden mit Ultraschall behandelt.

4. Messung von CRP-Konzentrationen in Serumproben

Das nach Beispiel 3 durch Bindung von Anti-CRP-Antikörpern an erfindungsgemäßen Latexpräparationen hergestellte Reagenz zur Bestimmung von CRP wurde zur Messung von CRP in Patientenseren eingesetzt. Als Standard wurde der LN-CRP-Standard (human) (Fa. Behringwerke AG) verwendet. Dieser (CRP-Standard enthielt laut Packungsbeilage 86 mg/l CRP. Der Standard wurde in Glycin-Kochsalz-Puffer (0,1 Mol Glycin, 0.17 M NaCl, pH 8,2) zunächst auf 2,3 mg/l verdünnt und dann stufenweise auf jeweils das doppelte Volumen weiterverdünnt. Man erhielt so eine Standardreihe mit abnehmenden CRP-Konzentrationen. Zur Messung wurden 10 µl Standardserumverdünnung mit 150 µl eines Reaktionspuffers (0,1 Mol Glycin, 0,17 Mol NaCl, 4 Gew. % Polyethylenglykol (PEG) 6000, 0,5 Gew. % Eikosaoxyethylen-sorbitanlaurylsäureester (Tween ® 20), pH 8,2 und mit 50 µl Reagenz nach Beispiel 3 in BLN-Küvetten (Fa. Behringwerke AG) gemischt und 30 min. bei Raumtemperatur inkubiert. Die Küvetten wurden dann im Lasernephelometer (Fa. Behringwerke AG) gemessen. Die Referenzkurve für die Messung der Standardseren wurde auf halblogarithmischem Papier gezeichnet und daran wurden die Meßwerte für die Patientenseren ausgewertet. Eine typische Referenzkurve ist in Fig. 2 dargestellt.

## Patentansprüche

1. Dispersionspolymere, dadurch gekennzeichnet, daß sie Latexpartikel enthalten mit einem Restmonomergehalt von 5 bis 50 Gew. %, bezogen auf die Latexpartikel, auf deren Oberfläche ein Copolymerisat aus einem Monomerengemisch sich befindet, das mindestens eine der Acetalgruppen enthaltenden Verbindungen der Formel I

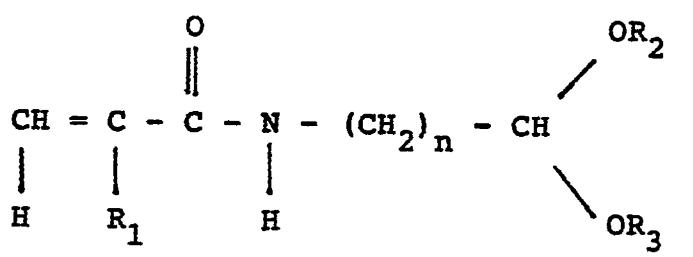

Formel I

worin n = 1 - 6;
$R_1$ = H, $CH_3$ und
$R_2$ und $R_3$ gleich oder unterschiedlich sind mit
$R_2$, $R_3$ = $-(CH_2)_m-CH_3$ m = 0 - 7 oder

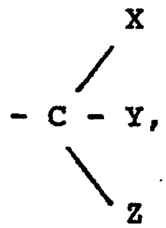

wobei X, Y, Z = $(CH_2)_pCH_3$ und p = 1 - 3 sind, mit X, Y, Z gleich oder unterschiedlich

oder $R_2$, $R_3$ = Arylrest

und gegebenenfalls Crotonsüure und/oder Acrylsäure und/oder Methacrylsäure und/oder carbocyclische, aromatische Monovinylidenmonomeren enthält.

2. Verfahren zur Herstellung von Dispersionspolymeren, dadurch gekennzeichnet, daß man zunächst Saatpolymere mit einem Restmonomergehalt von 5 bis 50 Gew. % herstellt, indem man die Polymerisation so durchführt, daß nur 30 bis 90 % des vorgegebenen Monomeren auspolymerisiert werden, sodann die nicht an das Polymer gebundenen Monomere abtrennt und anschließend in Anwesenheit des so erhaltenen Saatpolymeren ein Monomerengemisch polymerisiert, das enthält mindestens eine Verbindung der Formel I und zusätzlich 0-30 Gew. %, bezogen auf die Mischung, Acryl-, Methacryl- und/oder Crotonsäure und/oder weitere 0-30 Gew. %, wiederum bezogen auf die Gesamtmischung, carbocyclische aromatische Vinylidenmonomere.

3. Dispersionspolymere nach Anspruch 1, wobei das Saatpolymer Polystyrol-Latex ist.

4. Dispersionspolymere nach Anspruch 1, wobei das Saatpolymer einen Restmonomergehalt von 10 bis 40 Gew. %, bezogen auf das Polymere, aufweist.

5. Dispersionspolymere nach Anspruch 1, wobei als Monomere der Formel I Acryl- oder Methacrylamidoalkylaldehyd-di-alkyl-acetal mit Alkyl = $C_2$ bis $C_8$ eingesetzt sind.

6. Dispersionspolymere nach Anspruch 1, worin als Monomer der Formel I Methacrylamidoacetaldehyd-di-n-pentylacetal verwendet wird.

7. Biologisch aktive Dispersionspolymere, bestehend aus Dispersionspolymeren gemäß Anspruch 1 und daran gebundene Antikörper, Antigene oder Haptene.

8. Verwendung eines biologisch aktiven Dispersionspolymeren nach Anspruch 7 zum diagnostischen Nachweis von Antigenen, Antikörpern oder Haptenen.

9. Verwendung eines biologisch aktiven Dispersionspolymeren nach Anspruch 7 in nephelometrischem und turbidimetrischem Verfahren sowie für Partikelzählverfahren.

10. Diagnostisches Mittel, das biologisch aktive Dispersionspolymere enthält, die aus Dispersionspolymeren nach Anspruch 1 und daran gebundenen Antikörpern, Antigenen oder Haptenen bestehen.

**Claims**

1. A dispersion polymer which contains latex particles with a residual monomer content of 5 to 50% by weight, based on the latex particles, on the surface of which is a copolymer of a monomer mixture which contains at least one of the compounds of the formula I containing acetal groups

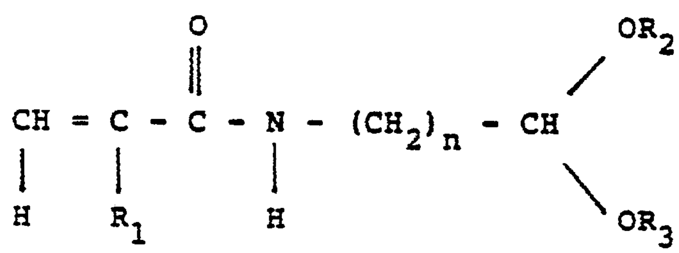

Formula I

where n = 1–6;
$R_1$ = H or $CH_3$ and

$R_2$ and $R_3$ are identical or different, where
$R_2$ and $R_3$ = $-(CH_2)_m-CH_3$, and m = 0–7, or

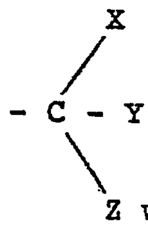
in which X, Y and Z = $(CH_2)_p-CH_3$ and p = 1 - 3,
where X, Y and Z are identical or different,
or $R_2$ and $R_3$ = an aryl radical,

and if appropriate crotonic acid and/or acrylic acid and/or methacrylic acid and/or carbocyclic aromatic monovinylidene monomers.

2. A process for the preparation of a dispersion polymer, which comprises first preparing a seed polymer with a residual monomer content of 5 to 50% by weight by carrying out the polymerization so that only 30 to 90% of the monomer present is polymerized completely, subsequently separating off the monomers not bonded to the polymer and then polymerizing a monomer mixture which contains at least one compound of the formula I and in addition 0–30% by weight, based on the mixture, of acrylic, methacrylic and/or crotonic acid and/or a further 0–30% by weight, again based on the total mixture, of carbocyclic aromatic vinylidene monomers in the presence of the seed polymer thus obtained.

3. A dispersion polymer as claimed in claim 1, in which the seed polymer is a polystyrene latex.

4. A dispersion polymer as claimed in claim 1, in which the seed polymer has a residual monomer content of 10 to 40% by weight, based on the polymer.

5. A dispersion polymer as claimed in claim 1, wherein an acryl- or methacrylamidoalkylaldehyde dialkyl acetal where alkyl = $C_2$ to $C_8$ is employed as the monomer of the formula I.

6. A dispersion polymer as claimed in claim 1, in which methacrylamidoacetaldehyde di-n-pentyl acetal is used as the monomer of the formula I.

7. A biologically active dispersion polymer consisting of a dispersion polymer as claimed in claim 1 and anti-bodies, antigens or haptens bound thereto.

8. The use of a biologically active dispersion polymer as claimed in claim 7 for diagnostic detection of antigens, antibodies or haptens.

9. The use of a biologically active dispersion polymer as claimed in claim 7 in a nephelometric or turbidometric method or for a particle counting method.

10. A diagnostic agent which contains a biologically active dispersion polymer which consists of a dispersion polymer as claimed in claim 1 and antibodies, antigens or haptens bound thereto.

## Revendications

1. Polymères en dispersion, caractérisés en ce qu'ils contiennent des particules de latex ayant une teneur en monomères résiduels de 5 à 50% en poids, par rapport aux particules de latex, à la surface desquelles se trouve un copolymère formé à partir d'un mélange de monomères qui contient au moins un des composés contenant des groupes acétal, de formule I

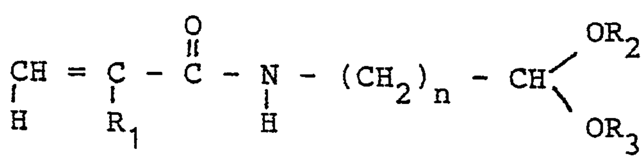
formule I

dans laquelle
n = 1–6;
$R_1$ = H, $CH_3$ et
$R_2$ et $R_3$ sont identiques ou différents, avec
$R_2$, $R_3$ = $-(CH_2)_m-CH_3$ m = 0–7, ou

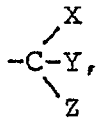 X, Y, Z étant $(CH_2)_p-CH_3$ et p = 1-3,
X, Y, Z étant identiques ou différents, ou

$R_2$, $R_3$ = radical aryle,

et éventuellement de l'acide crotonique et/ou de l'acide acrylique et/ou de l'acide méthacrylique et/ou des monomères monovinylidène aromatiques carbocycliques.

2. Procédé pour la préparation de polymères en dispersion, caractérisé en ce que l'on prépare d'abord des polymères d'ensemencement ayant une teneur en monomères résiduels de 5 à 50% en poids, en effectuant la polymérisation de manière que seulement de 30 à 90% du monomère disposé au préalable soient polymérisés, puis en séparant les monomères non liés au polymère et en polymérisant ensuite, en présence du polymère d'ensemencement ainsi obtenu, un mélange de monomères qui contient au moins un composé de formule I et en outre 0–30% en poids, par rapport au mélange, d'acide acrylique, méthacrylique et/ou crotonique et/ou de nouveau 0–30% en poids, là encore par rapport au mélange total, de monomères vinylidène aromatiques carbocycliques.

3. Polymères en dispersion selon la revendication 1, dans lesquels le polymère d'ensemencement est un latex de polystyrène.

4. Polymères en dispersion selon la revendication 1, dans lesquels le polymère d'ensemencement présente une teneur en monomères résiduels de 10 à 40% en poids, par rapport au polymère.

5. Polymères en dispersion selon la revendication 1, dans lesquels on utilise en tant que monomère de formule I un acryl- ou méthacrylamidoalkylaldéhyde-dialkylacétal dont les fragments alkyle sont en $C_2$ à $C_8$.

6. Polymères en dispersion selon la revendication 1, dans lesquels on utilise en tant que monomère de formule Ile méthacrylamidoacétaldéhyde-di-n-pentylacétal.

7. Polymères en dispersion biologiquement actifs, constitués de polymères en dispersion selon la revendication 1 et d'anticorps, antigènes ou haptènes liés à ceux-ci.

8. Utilisation d'un polymère en dispersion biologiquement actif selon la revendication 7, pour la détermination diagnostique d'antigènes, anticorps ou haptènes.

9. Utilisation d'un polymère en dispersion biologiquement actif selon la revendication 7, dans un procédé néphélométrique ou turbidimétrique ainsi que pour un procédé de comptage de particules.

10. Produit diagnostique contenant un polymère en dispersion biologiquement actif, qui consiste en des polymères en dispersion selon la revendication 1 et d'anticorps, antigènes ou haptènes liés à ceux-ci.

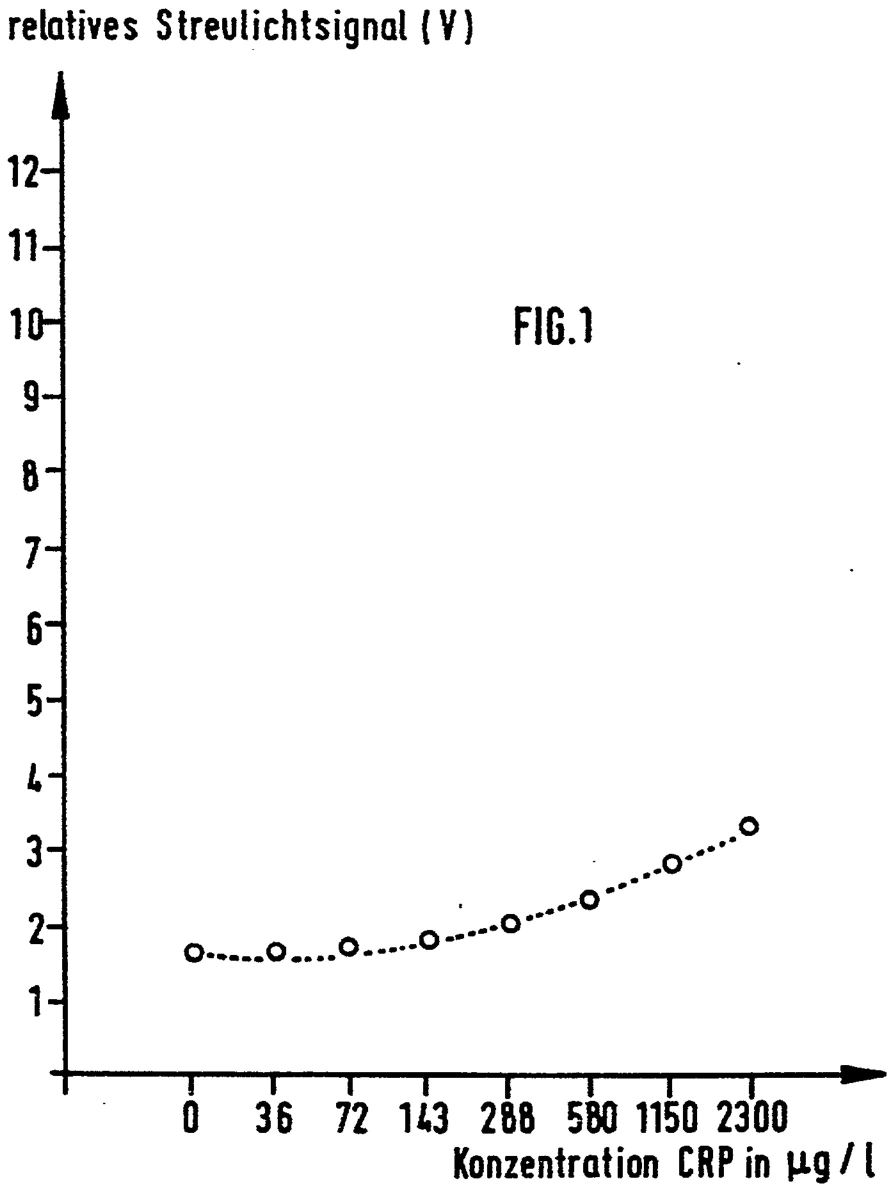
relatives Streulichtsignal (V)
12
11
10
9
8
7
6
5
4
3
2
1
0
36
72
143
288
580
1150
2300
Konzentration CRP in µg/l

FIG.1

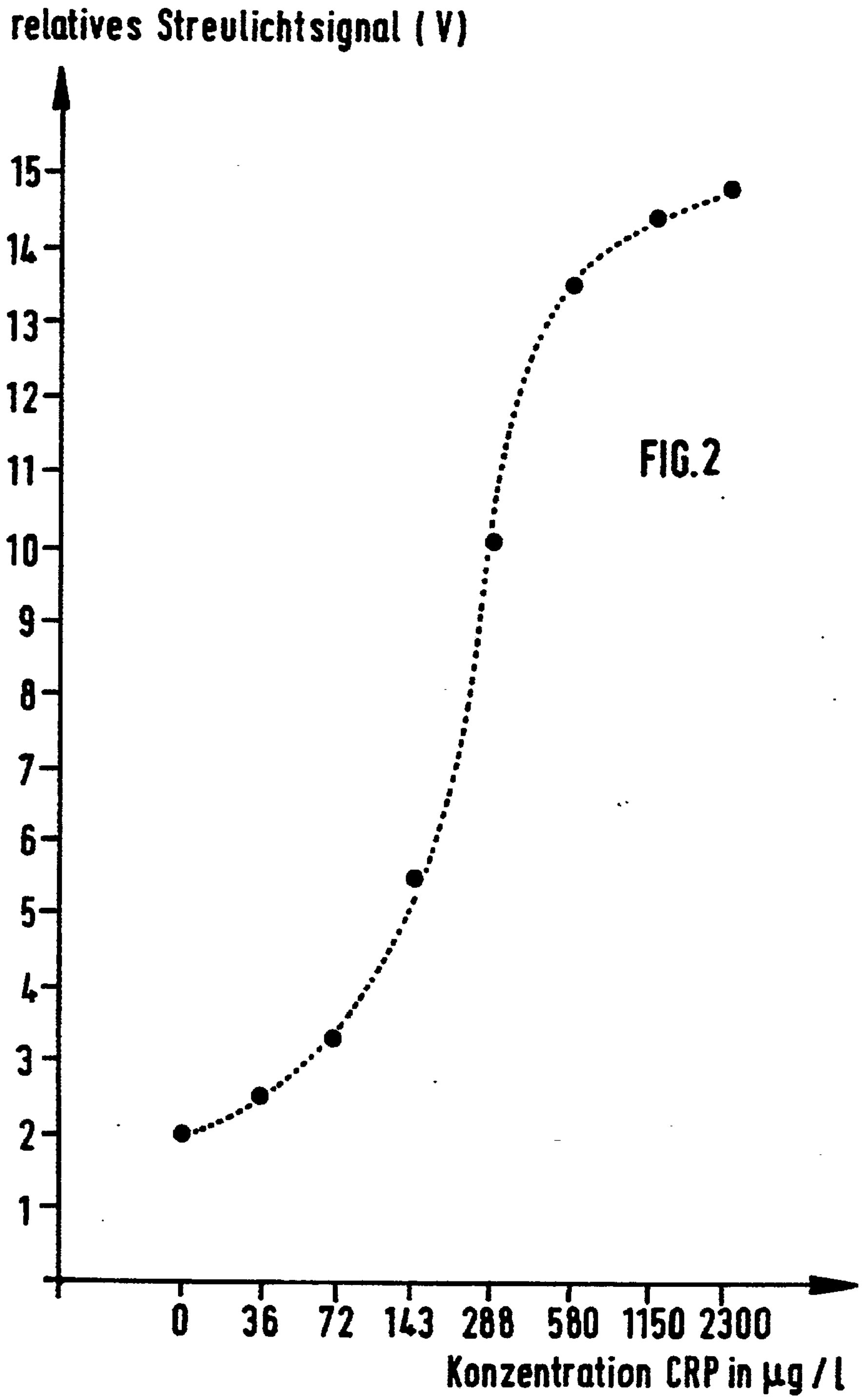
relatives Streulichtsignal ( V)
15
14
13
12
11
10
9
8
7
6
5
4
3
2
1
0
36
72
143
288
580
1150
2300
Konzentration CRP in µg / l

FIG.2